# EUROPÄISCHE PATENTANMELDUNG

(11) **EP 1 634 576 A1**
(43) Veröffentlichungstag der Anmeldung: **15.03.2006**
(21) Anmeldenummer: 05017347.5
(22) Anmeldetag: 10.08.2005
(51) Int. Cl.: A61K 8/64, A61K 8/66, A61Q 19/08

(54) **Kosmetische und dermatologische Zusammensetzungen mit DNA-Reparaturenzymen und Oligopeptiden**

(30) Priorität: 13.08.2004 DE 202004012807 U
(71) Anmelder: Henkel Kommanditgesellschaft auf Aktien, 40589 Düsseldorf (DE)
(72) Erfinder: Heinen, Soraya, 50858 Köln (DE)

(57) **Zusammenfassung**

Die Erfindung betrifft topische kosmetische oder dermatologische Zusammensetzungen, die in einem geeigneten kosmetischen oder dermatologischen Träger mindestens ein DNA-Reparaturenzym und mindestens ein ausgewähltes Oligopeptid enthalten.

## Beschreibung

Die Erfindung betrifft topische kosmetische oder dermatologische Zusammensetzungen, die in einem geeigneten kosmetischen oder dermatologischen Träger mindestens ein DNA-Reparaturenzym und mindestens ein ausgewähltes Oligopeptid enthalten.

Photolyase und T4 Endonuclease V, letztere im weiteren mit "T4N5" abgekürzt, sind im Stand der Technik bereits als sogenannte DNA-Reparatur-Enzyme bekannt. Unter DNA-Reparatur ist erfindungsgemäß die Spaltung bzw. Entfernung von UV-induzierten Pyrimidindimeren aus der DNA zu verstehen. "Pyrimidindimer" ist die im Stand der Technik gebräuchliche Bezeichnung für Dimere, die photochemisch, z. B. durch UV B-Strahlen, aus bestimmten Pyrimidinbasen der DNA gebildet werden. Pyrimidin selbst ist keine DNA-Base, dennoch wird im folgenden der Begriff "Pyrimidindimer" anstatt des korrekten Terminus "Pyrimidinbasen-Dimer" verwendet. Die Dimerisierung an der Pyrimidinbase Thymin erfolgt, indem benachbarte Thymin-Einheiten eines DNA-Stranges zu einer tricyclischen Verbindung dimerisieren. Das Dimerisierungsprodukt, eine cis-syn-Cyclobutandipyrimidin-Einheit, kann Fehler bei der Übertragung des genetischen Codes auslösen. Von der Bildung der Pyrimidindimeren sind vor allem die epidermalen Keratinozyten betroffen.

Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans*, einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.

Das Enzym T4 Endonuclease V wird vom *den*V-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.

DNA-Reparatur-Enzyme stellen interessante Wirkstoffe für kosmetische Zusammensetzungen dar. Bei den im Stand der Technik bevorzugten kosmetischen Zusammensetzungen handelt es sich um Sonnenschutzmittel und After-Sun-Produkte. Die Liposomenverkapselung von T4N5 wird von Ceccoli et al., J. Invest. Dermatol. 93, 190 - 194, 1989, beschrieben. Den Einsatz von liposomenverkapselter T4N5 bzw. Photolyase in kosmetischen Mitteln beschreiben Yarosh (US 5,190,762; WO 94/14419 A1) und Gilchrest et al. (WO 94/17781 A1). Burmeister et al. (EP 0 707 844 A2) offenbaren Zusammensetzungen, die liposomenverkapselte Kombinationen von DNA-Reparatur-Enzymen mit Tyrosin, Tyrosinderivaten, Vitaminen oder Provitaminen der Vitamin-Gruppen A, C und E, Glycoprotein-Komplexen von Kupfer, Zink oder Magnesium, Forskolin, cyclischem Adenosinmonophosphat (c-AMP), Bioflavonoiden oder Emulgatoren mit einem HLB-Wert von 10 - 14 enthalten, sowie Verfahren zur Herstellung von kosmetischen Bräunungsmitteln und Haarpflegeprodukten. In neueren Veröffentlichungen, insbesondere EP 1 153 600 A2, WO 02/49593 A2 und DE 203 16 269 U1, sind weitere kosmetische Zusammensetzungen mit DNA-Reparaturenzymen offenbart.

Es wurde festgestellt, dass die bekannten Zusammensetzungen bestimmte Alterungserscheinungen der Haut, insbesondere die lichtinduzierten Hautalterungserscheinungen wie Fältchen, nicht immer in zufriedenstellender Weise verbessern können.
Aufgabe der vorliegenden Erfindung war es daher, eine kosmetische oder dermatologische topische Zusammensetzung bereitzustellen, die eine verbesserte Behandlung von Fältchen, insbesondere von Fältchen, die auf die lichtinduzierte Hautalterung zurückzuführen sind, ermöglicht.
Eine weitere Aufgabe der vorliegenden Erfindung war es, eine kosmetische oder dermatologische topische Zusammensetzung bereitzustellen, die das Erscheinungsbild der Haut, insbesondere der lichtgealterten Haut, verbessert.
Überraschend wurde gefunden, dass kosmetische oder dermatologische topische Zusammensetzungen, die mindestens ein DNA-Reparaturenzym und mindestens ein Oligopeptid, das einen N-C₂-C₂₄-Acylrest aufweisen und/oder verestert sein kann, enthalten, in für den Fachmann nicht vorhersehbarer Weise die Nachteile des Standes der Technik beseitigen.

Gegenstand der vorliegenden Erfindung sind kosmetische oder dermatologische topische Zusammensetzungen, die in einem geeigneten kosmetischen oder dermatologischen Träger mindestens ein DNA-Reparaturenzym und mindestens ein Oligopeptid, das mindestens einen N-C₂-C₂₄-Acylrest aufweisen und/oder verestert sein kann, enthalten.

Bevorzugte erfindungsgemäße Zusammensetzungen sind dadurch gekennzeichnet, dass das DNA-Reparaturenzym ausgewählt ist aus Photolyase und T 4 Endonuclease V sowie Mischungen dieser Enzyme.

Die DNA-Reparaturenzyme können sowohl in Liposomen verkapselt als auch frei, das heißt unverkapselt, vorliegen. Die Liposomenverkapselung kann bevorzugt mit Phospholipiden, besonders bevorzugt mit Lecithin, erfolgen. Erfindungsgemäß besonders bevorzugt ist der Einsatz von mindestens einem liposomenverkapselten DNA-Reparaturenzym. Liposomenverkapselte Photolyase ist im Handel z. B. unter der Produktbezeichnung Photosomes™ (INCI-Bezeichnung: Aqua, Lecithin, Plankton Extract) liposomenverkapselte T4N5 z. B. unter der Bezeichnung Ultrasomes™ (INCI-Bezeichnung: Aqua, Lecithin, Micrococcus Lysate) von der Firma AGI Dermatics, USA, erhältlich. Da sich bei längerer Lagerung sowohl des Handelsproduktes als auch der erfindungsgemäßen Zusammensetzungen zwischen der verkapselten wässrigen, das Enzym enthaltenden Phase und der nicht-verkapselten, äußeren wässrigen Phase ein Gleichgewicht einstellt, liegt ein Teil der DNA-Reparaturenzyme unverkapselt vor.
In den erfindungsgemäßen Zusammensetzungen sind die Handelsprodukte Photosomes™ oder Ultrasomes™ bevorzugt in Mengen von 0,1 - 10 Gew.-%, besonders bevorzugt 0,5 - 5,0 Gew.-% und außerordentlich bevorzugt 1,0 - 4,0 Gew.-%, bezogen auf das gesamte Mittel, enthalten.
Bevorzugte erfindungsgemäße Zusammensetzungen sind dadurch gekennzeichnet, dass mindestens ein DNA-Reparaturenzym in Gesamtmengen von 0,00001 - 1 Gew.-%, bevorzugt 0,0001 - 0,1 Gew.-%, besonders bevorzugt 0,001 - 0,01 - 0,05 Gew.-% und außerordentlich bevorzugt 0,002 - 0,005 Gew.-%, enthalten ist.

Unter den in den erfindungsgemäßen Zusammensetzungen enthaltenen Oligopeptiden, die man auch als Aminosäureoligomere bezeichnen kann, werden erfindungsgemäß Peptide mit 2 - 30, bevorzugt 2 - 15, Aminosäuren, verstanden.
Die Aminosäuren und/oder die N-C₂-C₂₄-Acylaminosäuren sind erfindungsgemäß ausgewählt aus Alanin, Arginin, Asparagin, Asparaginsäure, Canavanin, Citrullin, Cystein, Cystin, Dipalmitoylhydroxyprolin, Desmosin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, Zink- und Natriumpyroglutamat und Natriumlauroylglutamat.
Der C₂ - C₂₄-Acylrest, mit dem die genannten Aminosäuren an der Aminogruppe derivatisiert sein können, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.
Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift PDHP von der Firma Seppic erhältlich ist.
Die physiologisch verträglichen Salze der erfindungsgemäß bevorzugten Oligopeptide, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.
Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere oder Oligopeptide stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z. B. die Collagensynthese, induzieren, beziehungsweise sind in der Lage, an die Sequenz Arg-Phe-Lys in Thrombospondin 1 (TSP-1) zu binden und damit aktives TGF-β *(tissue growth factor*), der die Synthese von Collagen in dermalen Fibroblasten induziert, freizusetzen. Derartige Aminosäureoligomere oder Oligopeptide können als Wirkstoffe gegen die Hautalterung verwendet werden.
Erfindungsgemäß bevorzugte, gegebenenfalls N-acylierte und/oder veresterte Dipeptide sind Acetyl-Citrullyl-Arginin (z. B. Exsy-Algine von Exsymol mit der INCI-Bezeichnung Acetyl Citrull Amido Arginine), Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester (z. B. Calmosensine von Sederma), Carnosin (β-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von der Firma Pentapharm unter der Bezeichnung SYN®-COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn N-Elaidoyl-Lys-Phe-Lys und N-Acetyl-Arg-Lys-Arg-NH2.
Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können.
Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile).
Die bevorzugten Aminosäuren, die Gln ersetzen können, beinhalten eine Seitenkette mit einer Aminogruppe, die bei neutralem pH (pH 6-7) überwiegend ungeladen vorliegt, z. B. Asn, Lys, Orn, 5-Hydroxyprolin, Citrullin und Canavanin.
Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.
Als Rigin-Analoga sind erfindungsgemäß Gly-Gln-Arg-Pro und Val-Val-Arg-Pro bevorzugt.
ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z. B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt.
Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von der Firma Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Ala-Asp-Leu-Lys-Pro-Thr (Hexapeptide-3, z. B. Peptide 02 von Vincience), Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Hexapeptide-11 (z. B. Peptamide-6 von Arch Personal Care), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), und Val-Val-Arg-Pro-Pro-Pro. Ein erfindungsgemäß bevorzugtes Pentadecapeptid ist z. B. der Rohstoff Vinci 01 von Vincience (Pentadecapeptide-1). Ein weiteres bevorzugtes Aminosäureoligomer ist das Peptidderivat L-Glutamylaminoethyl-indol (Glistin von Exsymol).
Es kann erfindungsgemäß besonders bevorzugt sein, Kombinationen aus mindestens zwei Oligopeptiden einzusetzen. Erfindungsgemäß besonders bevorzugt sind Kombinationen aus N-Palmitoyl-Gly-His-Lys und N-Palmitoyl-Gly-Gln-Pro-Arg, wie sie beispielsweise in dem Rohstoff Matrixyl 3000 von der Firma Sederma erhältlich sind.

Erfindungsgemäß bevorzugte Zusammensetzungen sind dadurch gekennzeichnet, dass sie mindestens ein Oligopeptid enthalten, das mindestens einen N-C₂-C₂₄-Acylrest aufweisen und/oder verestert sein kann und das ausgewählt ist aus Tyr-Arg und seinen N-acylierten Derivaten, insbesondere N-Acetyl-Tyr-Arg-hexyldecylester, Gly-His-Lys und seinen N-acylierten Derivaten, insbesondere N-Palmitoyl-Gly-His-Lys, Gly-His-Arg, und seinen N-acylierten Derivaten, insbesondere N-Myristoyl-Gly-His-Arg, Lys-Val-Lys und seinen N-acylierten Derivaten, insbesondere Palmitoyl-Lys-Val-Lys, Val-Tyr-Val, Gly-Gln-Pro-Arg (Rigin), Rigin-Analoga und Rigin-Derivaten, insbesondere N-Palmitoyl-Gly-Gln-Pro-Arg, Lys-Thr-Thr-Lys-Ser und seinen N-acylierten Derivaten, insbesondere N-Palmitoyl-Lys-Thr-Thr-Lys-Ser, Val-Gly-Val-Ala-Pro-Gly und seinen N-acylierten Derivaten, insbesondere N-Palmitoyl-Val-Gly-Val-Ala-Pro-Gly, Acetyl-Hexapeptide-3, Hexapeptide-4, Hexapeptide-5, Myristoyl Hexapeptide-5, Myristoyl Hexapeptide-6, Hexapeptide-8, Hexapeptide-9, Hexapeptide-10, L-Glutamylaminoethyl-indol, sowie Kombinationen dieser Substanzen, insbesondere Kombinationen aus N-Palmitoyl-Gly-His-Lys und N-Palmitoyl-Gly-Gln-Pro-Arg.

Besonders bevorzugte erfindungsgemäße Zusammensetzungen sind dadurch gekennzeichnet, dass mindestens ein Oligopeptid in Gesamtmengen von 0,000001 - 1 Gew.-%, bevorzugt 0,00001 - 0,1 Gew.-%, besonders bevorzugt 0,0001 - 0,001 - 0,01 - 0,05 Gew.-% und außerordentlich bevorzugt 0,002 - 0,005 Gew.-%, enthalten ist.

Weiterhin wurde festgestellt, dass sich die kosmetische Wirkung der erfindungsgemäßen Kombinationen aus DNA-Reparaturenzym und Oligopeptid weiter steigern lässt, wenn die erfindungsgemäßen Zusammensetzungen mindestens einen Wirkstoff, ausgewählt aus natürlichen Betainverbindungen, Vitaminen, Provitaminen und Vitaminvorstufen der Gruppen A, B, C, E, H und K und den Estern der vorgenannten Substanzen, den Flavonoiden Naringin, α-Glucosylrutin, α-Glucosylmyricetin, α-Glucosylisoquercetin, α-Glucosylquercetin, Dihydroquercetin (Taxifolin), Hesperidin, Hesperitin, Neohesperidin, Rutin, Troxerutin, Monoxerutin, Diosmin, Eriodictin und Apigenin-7-glucosid, Isoflavonoiden und Isoflavonoid-reichen Pflanzenextrakten, Polyphenolen und Polyphenol-reichen Pflanzenextrakten, Ubichinon und Ubichinol sowie deren Derivaten, Silymarin, natürlich vorkommenden Xanthin-Derivaten, ausgewählt aus Coffein, Theophyllin, Theobromin und Aminophyllin, Ectoin, Kreatin, Olivenblattextrakten, Ursolsäure, Oleanol und/oder Oleanolsäure, Mono- und Polyhydroxystilbenen und deren Estern, Derivaten von methyliertem Silanol, Phytinsäure, sowie Desoxyzuckern oder Desoxyzucker-Bausteine enthaltenden Polysacchariden, enthalten.
Bevorzugte erfindungsgemäße kosmetische oder dermatologische topische Zusammensetzungen sind dadurch gekennzeichnet, dass mindestens ein Wirkstoff, ausgewählt aus natürlichen Betainverbindungen, Vitaminen, Provitaminen und Vitaminvorstufen der Gruppen A, B, C, E, H und K und den Estern der vorgenannten Substanzen, den Flavonoiden Naringin, α-Glucosylrutin, α-Glucosylmyricetin, α-Glucosylisoquercetin, α-Glucosylquercetin, Dihydroquercetin (Taxifolin), Hesperidin, Hesperitin, Neohesperidin, Rutin, Troxerutin, Monoxerutin, Diosmin, Eriodictin und Apigenin-7-glucosid, Isoflavonoiden und Isoflavonoid-reichen Pflanzenextrakten, Polyphenolen und Polyphenol-reichen Pflanzenextrakten, Ubichinon und Ubichinol sowie deren Derivaten, Silymarin, natürlich vorkommenden Xanthin-Derivaten, ausgewählt aus Coffein, Theophyllin, Theobromin und Aminophyllin, Ectoin, Kreatin, Olivenblattextrakten, Ursolsäure, Oleanol und/oder Oleanolsäure, Mono- und Polyhydroxystilbenen und deren Estern, Derivaten von methyliertem Silanol, Phytinsäure, sowie Desoxyzuckern oder Desoxyzucker-Bausteine enthaltenden Polysacchariden, enthalten ist.
In einer bevorzugten Ausführungsform enthalten die erfindungsgemäßen Zusammensetzungen mindestens eine natürliche Betainverbindung. Erfindungsgemäße natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Bevorzugte natürliche Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂₋(im Falle des Carnitins).
Erfindungsgemäß besonders bevorzugte Zusammensetzungen sind dadurch gekennzeichnet, dass sie mindestens eine natürliche Betainverbindung in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.

In einer weiteren bevorzugten Ausführungsform enthalten die erfindungsgemäßen Zusammensetzungen mindestens ein Vitamin, Provitamin oder eine als Vitaminvorstufe bezeichnete Verbindung aus den Vitamingruppen A, B, C, E, H und K und den Estern der vorgenannten Substanzen.
Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente erfindungsgemäß besonders bevorzugt sind Vitamin A-Säure und deren Ester, Vitamin A-Aldehyd und Vitamin A-Alkohol sowie dessen Ester, wie Retinylpalmitat und Retinylacetat. Erfindungsgemäß besonders bevorzugte Zusammensetzungen sind dadurch gekennzeichnet, dass sie mindestens ein Vitamin, Provitamin oder eine als Vitaminvorstufe bezeichnete Verbindung aus der Vitamingruppe A oder mindestens einen Ester hiervon in Gesamtmengen von 0,001 - 2 Gew.-%, bevorzugt 0,05 - 0,05 - 1 Gew.-%, bezogen auf die gesamte Zusammensetzung, enthalten.
Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,0005 bis 0,1 - 1 Gew.-%, bezogen auf die gesamte erfindungsgemäße Zusammensetzung, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,0005 bis 0,1 - 1 Gew.-%, bezogen auf die gesamte erfindungsgemäße Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,0005 bis 0,1 - 1 Gew.-%, bezogen auf die gesamte erfindungsgemäße Zusammensetzung, enthalten ist.
- Vitamin B₅ (Pantothensäure und Panthenol). Bevorzugt wird Panthenol eingesetzt. Erfindungsgemäß bevorzugte Derivate des Panthenols sind insbesondere die Ester und Ether des Panthenols sowie kationisch derivatisierte Panthenole. In einer weiteren bevorzugten Ausführungsform der Erfindung werden an Stelle von sowie zusätzlich zu Pantothensäure oder Panthenol auch Derivate des 2-Furanon mit der allgemeinen Strukturformel (I) eingesetzt.

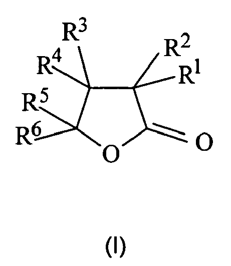

Besonders bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
Erfindungsgemäß besonders bevorzugte Zusammensetzungen sind dadurch gekennzeichnet, dass sie mindestens eine der genannten Verbindungen des Vitamin B₅-Typs sowie der 2-Furanonderivate in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Erfindungsgemäß besonders bevorzugte Zusammensetzungen sind dadurch gekennzeichnet, dass sie mindestens eine Vitamin B₆-Komponente in einer Gesamtmenge von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Erfindungsgemäß besonders bevorzugte Zusammensetzungen sind dadurch gekennzeichnet, dass sie mindestens eine Komponente, ausgewählt aus Biotin und den Biotinestern, in einer Gesamtmenge von 0,0001 bis 1,0 Gew.-%, insbesondere 0,001 bis 0,01 Gew.-%, enthalten.
In einer weiteren bevorzugten Ausführungsform enthalten die erfindungsgemäßen Zusammensetzungen mindestens eine Substanz, die ausgewählt ist aus den Vitaminen, Provitaminen und Vitaminvorstufen der Gruppe B₁, B₂, B₃, B₆, B₇ und deren Estern und aus Pantolacton.

Bevorzugte Vitamine, Provitamine und Vitaminvorstufen der Gruppe C und deren Ester sind Vitamin C (Ascorbinsäure) und die Derivate Ascorbylpalmitat, -stearat, -dipalmitat, -acetat, Magnesiumascorbylphosphat, Natriumascorbylphosphat, Natrium- und Magnesiumascorbat, Dinatriumascorbylphosphat und -sulfat, Kaliumascorbyltocopherylphosphat, Chitosanascorbat oder Ascorbylglucosid. Die Kombination mit Tocopherolen kann ebenfalls bevorzugt sein. Erfindungsgemäß besonders bevorzugte Zusammensetzungen sind dadurch gekennzeichnet, dass sie mindestens eine der genannten Verbindungen des Vitamin C-Typs in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.
Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Erfindungsgemäß besonders bevorzugte Zusammensetzungen sind dadurch gekennzeichnet, dass sie mindestens eine Substanz, ausgewählt aus Tocopherol und seinen Derivaten, in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.

Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Erfindungsgemäß besonders bevorzugte Zusammensetzungen sind dadurch gekennzeichnet, dass sie mindestens ein Vitamin K in einer Gesamtmenge von 0001 bis 1,0 Gew.-%, bevorzugt 0,05 bis 0,01 Gew.-%, besonders bevorzugt 0,1 bis 0,5 Gew.-%, jeweils bezogen auf die gesamte Zusammensetzung, enthalten.

Vitamin A-palmitat (Retinylpalmitat), Panthenol, Pantolacton, Nicotinsäureamid, Pyridoxin, Pyridoxamin, Pyridoxal, Biotin, Ascorbylpalmitat, Ascorbylacetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

In einer weiteren bevorzugten Ausführungsform enthalten die erfindungsgemäßen Zusammensetzungen mindestens ein Flavonoid oder mindestens einen Flavonoidreichen Pflanzenextrakt.
Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Dihydroquercetin (Taxifolin), Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid).
Erfindungsgemäß außerordentlich bevorzugte Flavonoide sind α-Glucosylrutin, Naringin und Apigenin-7-glucosid.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon.
Erfindungsgemäß besonders bevorzugte Zusammensetzungen sind dadurch gekennzeichnet, dass sie mindestens ein Flavonoid in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Isoflavonoid oder mindestens einen Isoflavonoid-reichen Pflanzenextrakt. Zu den Isoflavonoiden werden an dieser Stelle die Isoflavone und die Isoflavon-Glycoside gezählt.
Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.
In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.
Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.
Erfindungsgemäß besonders bevorzugte Zusammensetzungen sind dadurch gekennzeichnet, dass sie mindestens ein Isoflavonoid in einer Gesamtmenge von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Polyphenol oder einen Polyphenol-reichen Pflanzenextrakt.
Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.
Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay L, ein Extrakt aus den Kernen der Chardonnay-Traube, erhältlich von der Firma Croda.
Erfindungsgemäß bevorzugt werden die Polyphenole in Mengen von 0,001 bis 10 Gew.-%, besonders bevorzugt 0,005 bis 5 Gew.-% und außerordentlich bevorzugt 0,01 bis 3 Gew.-%, jeweils bezogen auf das Gewicht des Handelsproduktes, das mindestens ein Polyphenol enthält, in der gesamten erfindungsgemäßen Zusammensetzung, eingesetzt.

In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinole sind die reduzierte Form der Ubichinone. Die erfindungsgemäß bevorzugten Ubichinone weisen die Formel (II) auf: 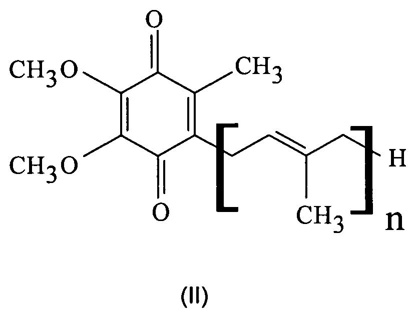 mit n = 6, 7, 8, 9 oder 10.
Besonders bevorzugt ist das Ubichinon der Formel (II) mit n = 10, auch bekannt als Coenzym Q10.

Erfindungsgemäß besonders bevorzugte Zusammensetzungen sind dadurch gekennzeichnet, dass sie mindestens ein Ubichinon, Ubichinol oder ein Derivat hiervon in einer Gesamtmenge von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Silymarin. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören. Erfindungsgemäß besonders bevorzugte Zusammensetzungen sind dadurch gekennzeichnet, dass sie Silymarin in Mengen von 0,00001 bis 1 Gew.-%, bevorzugt 0,0001 bis 0,01 Gew.-% und besonders bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen Kreatin. Kreatin ist der Trivialname für *N*-Methyl-guanidino-essigsäure bzw. N-Amidinosarkosin. Erfindungsgemäß bevorzugt ist Kreatin in Mengen von 0,0001 bis 1 Gew.-%, besonders bevorzugt 0,001 bis 0,5 Gew.-% und außerordentlich bevorzugt 0,01 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens einen Olivenblattextrakt (Olea Europaea (Olive) Leaf Extract). Ein erfindungsgemäß besonders bevorzugter Olivenblattextrakt ist unter der Handelsbezeichnung Oleanoline DPG von der Firma Vincience erhältlich. Ein weiterer erfindungsgemäß besonders bevorzugter Olivenblattextrakt ist unter der Handelsbezeichnung Olea europ Fol extr. S. sicc. von der Firma Fruitarom erhältlich.
Erfindungsgemäß besonders bevorzugte Zusammensetzungen sind dadurch gekennzeichnet, dass sie mindestens einen Olivenblattextrakt in einer Gesamtmenge von 0,01 bis 5 Gew.-%, bevorzugt 0,1 bis 3 Gew.-% und besonders bevorzugt 0,5 bis 1 - 2 Gew.-%, jeweils bezogen auf den Extrakt als Handelsprodukt tel quel in der gesamten erfindungsgemäßen Zusammensetzung, enthalten.

Olivenblattextrakte können einen hohen Gehalt an Oleanolsäure und/oder Oleanol aufweisen. In einer weiteren bevorzugten Ausführungsform enthalten die erfindungsgemäßen Zusammensetzungen Oleanolsäure und/oder Oleanol. Erfindungsgemäß besonders bevorzugte Zusammensetzungen sind dadurch gekennzeichnet, dass sie Oleanolsäure und/oder Oleanol in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Zusammensetzung, enthalten.

In einer weiteren bevorzugten Ausführungsform enthalten die erfindungsgemäßen Zusammensetzungen Ursolsäure. Erfindungsgemäß besonders bevorzugte Zusammensetzungen sind dadurch gekennzeichnet, dass sie Ursolsäure in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Zusammensetzung, enthalten.
In einer weiteren bevorzugten Ausführungsform enthalten die erfindungsgemäßen Zusammensetzungen mindestens einen Wirkstoff, der ausgewählt ist aus den Mono- und Polyhydroxystilbenen und deren Estern. Unter Polyhydroxystilbenen werden erfindungsgemäß Stilbene verstanden, die mit 2, 3, 4, 5, 6, 7, 8, 9 oder 10 Hydroxygruppen an den beiden Phenylresten substituiert sind, wobei diese verestert sein können. Mono- und Polyhydroxystilbene und deren Ester erhöhen und/oder verbessern die Interaktion zwischen der extrazellulären Matrix und den Fibroblasten. Erfindungsgemäß besonders bevorzugte Hydroxystilbene und deren Ester sind ausgewählt aus Resveratrol (trans-Stilben-3,4'-5-triol), den Resveratrolmono-, -di- und -triphosphorsäureestern und deren Salzen. Ein erfindungsgemäß besonders bevorzugter Resveratrolphosphorsäureester ist Trisodium Resveratrol Triphosphate, z. B. erhältlich von Ajinomoto.
Erfindungsgemäß besonders bevorzugte kosmetische oder dermatologische Zusammensetzungen sind dadurch gekennzeichnet, dass sie mindestens einen Wirkstoff, der ausgewählt ist aus den Mono- und Polyhydroxystilbenen und deren Estern, in einer Gesamtmenge von 0,000001 - 5 Gew.-%, bevorzugt 0,00001 - 1 Gew.-%, besonders bevorzugt 0,0001 - 0,1 Gew.-% und außerordentlich bevorzugt 0,005 - 0,05 Gew.-%, enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens ein Derivat von methyliertem Silanol, vorzugsweise mindestens einen Ester von methyliertem Silanol. Bevorzugte Derivate von methyliertem Silanol sind ausgewählt aus:
- sodium mannuronate methylsilanol (Algisium, Exsymol)
- methylsilanol mannuronate (Algisium C®, Exsymol)
- methylsilanol mannuronate Nylon-12 (Algisium C powder®, Exsymol)
- ascorbylmethylsilanol (Ascorbosilane concentrate C®, Exsymol)
- ascorbylmethylsilanol pectinate (Ascorbosilane C®, Exsymol)
- dimethyl oxobenzodioxsilane (DSBC®, Exsymol)
- dimethyl oxobenzodioxasilane Nylon-12 (DSBC powder®, Exsymol)
- sodium hyaluronate dimethylsilanol (DSH®, Exsymol)
- dimethylsilanol hyaluronate (DSHC®, Exsymol)
- methysilanol glycyrrhizinate (Glysinol®, Exsymol)
- methylsilanolhydroxyproline (Hydroxyprolisilane®, Exsymol)
- methylsilanolhydroxyproline aspartate (Hydroxyprolisilane C®, Exsymol)
- sodium lactate methylsilanol (Lasilium®, Exsymol)
- lactoylmethylsilanol elastinate (Lasilium C®, Exsymol)
- dioleyl tocopheryl methylsilanol (Liposiliol C®, Exsymol)
- methylsilanol acetylmethionate (Methiosilane®, Exsymol)
- acetylmethionylmethylsifanol elastinate (Methiosilane C®, Exsymol)
- methylsilanol PEG 7 glyceryl cocoate (Monosiliol®, Exsymol)
- methylsilanol tri PEG 7 glyceryl cocoate (Monosiliol C®, Exsymol)
- methylsilanol elastinate (Proteosilane C®, Exsymol)
- pyrollidone carboxylate caustic methylsilanol (Silhydrate®, Exsymol)
- pyrollidone carboxylate copper methylsilanol (Silhydrate C®, Exsymol)
- methylsilanolcarboxymethyl theophylline (Theophyllisilane®, Exsymol)
- methylsilancarboxymethyl theophylline alginate (Theophyllisilane C® Exsymol)
- methylsilanol acetyltyrosine (Tyrosilane®, Exsymol)
- copper acetyl tyrosinate methylsilanol (Tyrosilane C®, Exsymol).
Besonders bevorzugt sind Sodium Hyaluronate Dimethylsilanol, Dimethylsilanol Hyaluronate, Methylsilanol Mannuronate, Methylsilanol Hydroxyproline und Methylsilanol Hydroxyproline Aspartate. In einer weiteren bevorzugten Ausführungsform enthalten die erfindungsgemäßen Zusammensetzungen mindestens ein Derivat von methyliertem Silanol in Gesamtmengen von 0,001 - 5 Gew.-%, bevorzugt 0,005 - 1 Gew.-% und besonders bevorzugt 0,01 - 0,5 Gew.-%, jeweils bezogen auf die Aktivsubstanz in der gesamten erfindungsgemäßen Zusammensetzung.

In einer weiteren bevorzugten Ausführungsform enthalten die erfindungsgemäßen Zusammensetzungen Phytinsäure. Erfindungsgemäß besonders bevorzugte kosmetische oder dermatologische Zusammensetzungen sind dadurch gekennzeichnet, dass sie Phytinsäure in einer Gesamtmenge von 0,001 - 1 Gew.-%, bevorzugt 0,01 - 0,5 Gew.-% und besonders bevorzugt 0,05 - 0,1 Gew.-% enthalten, jeweils bezogen auf die gesamte Zusammensetzung.

In einer weiteren bevorzugten Ausführungsform enthalten die erfindungsgemäßen Zusammensetzungen mindestens einen Desoxyzucker oder ein mindestens einen Desoxyzucker-Baustein enthaltendes Polysaccharid.
Erfindungsgemäß bevorzugte Desoxyzucker sind ausgewählt aus Rhamnose und Fucose. Ein erfindungsgemäß besonders bevorzugtes, Desoxyzucker-Bausteine enthaltendes Polysaccharid ist das Handelsprodukt Fucogel® von Solabia mit der INCI-Bezeichnung Biosaccharide Gum-1. Ein weiteres erfindungsgemäß besonders bevorzugtes, Desoxyzucker-Bausteine enthaltendes Polysaccharid ist das Handelsprodukt Rhamnosoft® von Solabia mit der INCI-Bezeichnung Biosaccharide Gum-2. Ein erfindungsgemäß besonders bevorzugtes, Desoxyzucker-Bausteine enthaltendes Polysaccharid ist das Handelsprodukt Fucogenol® von Solabia mit der INCI-Bezeichnung Biosaccharide Gum-3. Ein erfindungsgemäß besonders bevorzugtes, Desoxyzucker-Bausteine enthaltendes Polysaccharid ist das Handelsprodukt Glycofilm® von Solabia mit der INCI-Bezeichnung Biosaccharide Gum-4. Erfindungsgemäß bevorzugt sind weiterhin Mischungen der vorgenannten, mindestens einen Desoxyzucker-Baustein enthaltenden Polysaccharide, beispielsweise die Mischung aus Biosaccharide Gum-2 und Biosaccharide Gum-3, erhältlich als Handelsprodukt Elastinol plus® von Solabia.
Erfindungsgemäß besonders bevorzugte kosmetische oder dermatologische Zusammensetzungen sind dadurch gekennzeichnet, dass sie mindestens einen Desoxyzucker und/oder mindestens ein Desoxyzucker-Bausteine enthaltendes Polysaccharid in Gesamtmengen von 0,001 bis 5 Gew.-%, bevorzugt 0,01 bis 2 Gew.-% und besonders bevorzugt 0,1 bis 1 Gew.-%, jeweils bezogen auf die gesamte erfindungsgemäße Zusammensetzung, enthalten.

Die erfindungsgemäßen kosmetischen oder dermatologischen topischen Zusammensetzungen, die mindestens ein DNA-Reparaturenzym und mindestens ein Oligopeptid, das einen N-C₂-C₂₄-Acylrest aufweisen und/oder verestert sein kann, enthalten, sind hervorragend zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut geeignet.
Ein weiterer Gegenstand der vorliegenden Erfindung ist daher die Verwendung einer kosmetischen oder dermatologischen topischen Zusammensetzung, die mindestens ein DNA-Reparaturenzym und mindestens ein Oligopeptid, das einen N-C₂-C₂₄-Acylrest aufweisen und/oder verestert sein kann, enthält, zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut.
Ein weiterer Gegenstand der vorliegenden Erfindung ist ein nicht-therapeutisches Verfahren zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut, das dadurch gekennzeichnet ist, dass eine kosmetische oder dermatologische topische Zusammensetzung, die mindestens ein DNA-Reparaturenzym und mindestens ein Oligopeptid, das einen N-C₂-C₂₄-Acylrest aufweisen und/oder verestert sein kann, enthält, auf die Haut aufgetragen wird.

Bevorzugt liegen die erfindungsgemäßen Hautbehandlungsmittel in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels oder Oleogels, einer ein- oder mehrphasigen Lösung, eines Schaumes, eines Puders oder einer Mischung mit mindestens einem als medizinischer Klebstoff geeigneten Polymer vor. Die Zusammensetzungen können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.
In einer besonders bevorzugten Ausführungsform liegen die erfindungsgemäßen Zusammensetzungen als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den drei Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen. Die DNA-Reparaturenzyme werden bevorzugt bei allen Herstellverfahren, die ein Erhitzen erfordern, erst nach dem Erhitzen in die abgekühlte Formulierung eingearbeitet.
In der Ausführungsform als Emulsion oder als tensidische Lösung, z. B. als Reinigungsmittel, enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov®68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospholipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls® PGPH) und Polyglyceryl-3-diisostearat (Handelsprodukt Lameform® TGI) sowie lineare und verzweigte C₈-C₃₀-Fettsäuren und deren Na-, K-, Ammonium-, Ca-, Mg- und Zn - Salze.
Die erfindungsgemäßen Zusammensetzungen enthalten die Emulgatoren bevorzugt in Mengen von 0,1 bis 25 Gew.-%, insbesondere 0,5 - 15 Gew.-%, bezogen auf die gesamte Zusammensetzung.
In einer weiteren besonders bevorzugten Ausführungsform ist mindestens ein nichtionischer Emulgator mit einem HLB-Wert von 8 und darunter enthalten. Derart geeignete Emulgatoren sind besonders bevorzugt Verbindungen der allgemeinen Formel R¹ - O - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen ist. Besonders bevorzugte Emulgatoren sind Behenylalkohol und Arachidylalkohol. Derartige Emulgatoren bilden sogenannte Lamellaremulsionen, deren Emulsionströpfchen von einer flüssig-kristallinen, lamellaren Phase aus Lipidmolekülen und Wasser umgeben und dadurch besonders stabilisiert und zur Wiederherstellung des gestörten Ordnungsgrades geschädigter Haut besonders geeignet sind. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythrit-monoerucat.
Besonders bevorzugte erfindungsgemäße Zusammensetzungen sind dadurch gekennzeichnet, dass mindestens ein nichtionischer Emulgator mit einem HLB-Wert von 8 und darunter mit der allgemeinen Formel R¹ - O - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen ist, in Gesamtmengen von 0,5 - 10 Gew.-%, bevorzugt 1 - 6 Gew.-% und besonders bevorzugt 2 - 4 Gew.-%, enthalten ist.
Weitere geeignete Zusatzstoffe sind Fettstoffe, insbesondere pflanzliche Öle, wie Olivenöl, Sonnenblumenöl, Sojaöl, Rapsöl, Mandelöl, Jojobaöl, Orangenöl, Weizenkeimöl, Pfirsichkernöl und die flüssigen Anteile des Kokosöls, flüssige Paraffinöle, Isoparaffinöle und synthetische Kohlenwasserstoffe, Di-n-alkylether mit insgesamt 12 bis 36 C-Atomen, z. B. Di-n-octylether und n-Hexyl-n-octylether, Fettsäuren, besonders lineare und/oder verzweigte, gesättigte und/oder ungesättigte C₈₋₃₀-Fettsäuren, Fettalkohole, besonders gesättigte, ein- oder mehrfach ungesättigte, verzweigte oder unverzweigte Fettalkohole mit 6 - 30 Kohlenstoffatomen, Esteröle, das heißt Ester von C₆₋₃₀-Fettsäuren mit C₂₋₃₀₋Fettalkoholen, Hydroxycarbonsäurealkylester, Dicarbonsäureester wie Di-n-butyladipat sowie Diolester wie Ethylenglykoldioleat oder Propylenglykoldi(2-ethylhexanoat), symmetrische, unsymmetrische oder cyclische Ester der Kohlensäure mit Fettalkoholen, z. B. Glycerincarbonat oder Dicaprylylcarbonat (Cetiol® CC), Mono,- Di- und Trifettsäureester von gesättigten und/oder ungesättigten linearen und/oder verzweigten Fettsäuren mit Glycerin Wachse, insbesondere Insektenwachse, Pflanzenwachse, Fruchtwachse, Ozokerit, Mikrowachse, Ceresin, Paraffinwachse, Triglyceride gesättigter und gegebenenfalls hydroxylierter C₁₆₋₃₀-Fettsäuren, z. B. gehärtete Triglyceridfette, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxane, Polyalkylarylsiloxane, ethoxylierte Polydialkylsiloxane sowie Polydialkylsiloxane, die Amin- und/oder Hydroxy-Gruppen enthalten.
In einer bevorzugten Ausführungsform der Erfindung beträgt die Einsatzmenge der Fettstoffe 0,1 - 50 Gew.-%, bevorzugt 0,1 - 20 Gew.-% und besonders bevorzugt 0,1 - 15 Gew.-%, jeweils bezogen auf die gesamte Zusammensetzung.
Weitere geeignete Zusatzstoffe sind Verdickungsmittel, z. B. natürliche und synthetische Tone und Schichtsilikate wie Bentonit, Hectorit, Montmorillonit oder Laponite®, oder anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel®305, Simulgel®600, Simulgel® NS, Simulgel® EPG und Simulgel® EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol®. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀₋Carbonsäuren, wobei das Copolymer mit den vorgenannten Vernetzungsagentien vernetzt sein kann. Entsprechende Handelsprodukte sind Pemulen® und die Carbopol®-Typen 954, 980, 1342 und ETD 2020 (ex B. F. Goodrich).
Geeignete nichtionische Polymere sind beispielsweise Polyvinylalkohole, die teilverseift sein können, z. B. die Handelsprodukte Mowiol® sowie VinylpyrrolidonNinylester-Copolymere und Polyvinylpyrrolidone, die z. B. unter dem Warenzeichen Luviskol® (BASF) vertrieben werden.
Weitere geeignete Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum®, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Trübungsmittel und Perlglanzmittel wie Ethylenglykolmono- und -distearat und Treibmittel wie Propan-Butan-Gemische, N₂O, Dimethylether, CO₂ und Luft.

Die nachfolgenden Beispiele sollen den Gegenstand der Erfindung erläutern, ohne ihn hierauf zu beschränken.

| | Tagescreme | Nachtcreme | Stiftfüllung | Tagescreme |
|---|---|---|---|---|
| Wässrige Lecithin-Liposomenzubereitung | 1,0000 | - | 1,0000 | 1,0000 |
| Isopropylstearat | 2,0000 | - | 2,0000 | 2,0000 |
| Montanov 68 | - | 3,0000 | - | - |
| Capryl-/Caprinsäure-Triglycerid | - | 5,0000 | - | - |
| Cetiol SN | 2,0000 | 3,0000 | - | 2,0000 |
| Safloröl raffiniert | 1,0000 | 5,0000 | 3,000 | 1,0000 |
| Cetiol SB 45 | - | 2,0000 | - | - |
| Cetiol B | 3,0000 | - | 1,5000 | 3,0000 |
| Vitamin E Acetat | 1,0000 | - | 1,0000 | 1,0000 |
| Cutina MD | 2,0000 | 1,0000 | 0,5000 | 2,0000 |
| Cetearyl Alcohol | 0,5000 | 1,5000 | 0,5000 | 0,5000 |
| NOVATA AB | 1,0000 | 2,5000 | 1,0000 | 1,0000 |
| Behenylalkohol | 1,5000 | 1,0000 | 0,5000 | 1,5000 |
| Dimethicone | 2,0000 | 1,5000 | 0,5000 | 2,0000 |
| Propylparaben | 0,2000 | 0,2000 | 0,2000 | 0,2000 |
| Dow Corning 9040 | 2,0000 | - | 2,0000 | 2,0000 |
| Talkum | 1,0000 | - | 0,5000 | 1,0000 |
| Dry Flo Plus | - | 1,0000 | - | - |
| Titandioxid | 0,1000 | 0,1000 | 0,1000 | 0,1000 |
| Glycerin 86% | 8,0000 | 5,0000 | 6,0000 | 8,0000 |
| Hexandiol-1,6 | 6,0000 | 4,0000 | 10,0000 | 6,0000 |
| Propandiol-1,2 | 2,0000 | - | 5,0000 | 2,0000 |
| Methylparaben | 0,2000 | 0,2000 | 0,2000 | 0,2000 |
| Tego Carbomer 140 | 0,2000 | 0,5000 | 0,2000 | 0,2000 |
| DSH-C N | 5,0000 | 2,0000 | 5,0000 | - |
| Photosomes | 1,0000 | - | 1,0000 | 1,0000 |
| Ultrasomes | - | 1,0000 | - | - |
| Lipochroman-6 | 0,0100 | - | 0,0100 | 0,0100 |
| Ederline L | - | 4,0000 | 2,0000 | - |
| Controx KS | - | 0,0500 | - | - |
| Matrixyl 3000 | 5,0000 | - | 5,0000 | 5,0000 |
| Matrixyl | - | 3,0000 | - | - |
| Biopeptide CL | - | 1,0000 | 2,0000 | - |
| Eyeliss | - | - | 4,0000 | - |
| α-Glucosylrutin | - | - | - | 0,0100 |
| Coenzym Q 10 | 0,0100 | - | - | 0,0100 |
| Carnitin | - | - | - | 0,5000 |
| Sepivinol | 0,5000 | - | - | - |
| Crodarom Chardonnay | - | - | - | 0,5000 |
| Phytokine | 1,0000 | - | - | - |
| Ridulisse C | - | - | - | 1,0000 |
| Phenoxyethanol | 0,2000 | 0,2000 | 0,2000 | 0,2000 |
| Ronasphere LDP | 2,0000 | - | 2,0000 | 2,0000 |
| Simulgel NS | 2,0000 | 0,5000 | 2,0000 | 2,0000 |
| Natriumhydroxid | 0,0220 | 0,0100 | 0,0220 | 0,0220 |
| WASSER VOLLENTSALZT | ad 100 | ad 100 | ad 100 | ad 100 |

### Zur Tabelle: Alle Mengenangaben sind in Gew.-%.

Die Stiftfüllungsrezeptur wurde in einen Flockstift-Applikator der Firma Schwan-Stabilo abgefüllt, der zur gezielten und sparsamen Applikation auf ausgewählte Problemzonen der Haut, beispielsweise auf Fältchen im Augen- oder Mundbereich, hervorragend geeignet ist. Erfindungsgemäß bevorzugte Flockstifte, die mit der erfindungsgemäßen Stiftfüllungsrezeptur befüllt wurden, sind in DE 198 58 410 A1 (insbesondere Figur 10), EP 1 010 379 A1 (insbesondere Figur 1), US 6,309,128 (insbesondere Figur 10), US 6,238,117 (insbesondere Figur 1), und DE 202 04 111 U (insbesondere Figur 3), beschrieben, deren Offenbarungsgehalt ausdrücklich Teil der vorliegenden Anmeldung ist.

### Liste der verwendeten Rohstoffe

| | |
|---|---|
| Biopeptide CL (Sederma) | N-Palmitoyl-Gly-His-Lys |
| Cetiol B (Cognis) | Diisopropyladipat |
| Cetiol SB 45 (Cognis) | Butyrospermum Parkii (Linne) |
| Cetiol SN (Cognis) | CETEARYL ISONONANOATE |
| Controx KS (Cognis) | Tocopherol, Hydrogenated Palm Glycerides Citrate |
| Crodarom Chardonnay L (Croda) | Propylene Glycol, Water, Vitis Vinifera (Grape) Seed Extract |
| Cutina MD (Cognis) | Glyceryl Stearate |
| Dow Corning 9040 | Cyclomethicone and Dimethicone Crosspolymer |
| Dry Flo Plus | Aluminium Starch Octenylsuccinate |
| DSH-C N (Exsymol) | Water, Dimethylsilanol-Hyaluronate |
| Ederline L | Hexyldecanol, Pyrus Malus (Apple) Fruit Extract |
| Eyeliss (Sederma) | Water, Glycerin, Hesperidin Methyl Chalcone, Steareth-20, Dipeptide-2, PALMITOYL TETRAPEPTIDE-3 |
| Lipochroman-6 | Dimethylmethoxy Chromanol |
| Matrixyl (Sederma) | GLYCERIN, BUTYLENE GLYCOL, AQUA (WATER), CARBOMER, POLYSORBATE-20, PALMITOYL-PENTAPEPTIDE-3 |
| Matrixyl 3000 (Sederma) | GLYCERIN, AQUA (WATER), BUTYLENE GLYCOL, CARBOMER, POLYSORBATE-20, PALMITOYL OLIGOPEPTIDE, PALMITOYL TETRAPEPTIDE-3 |
| Montanov 68 (Seppic) | Cetearyl Alcohol, Cetearyl Glucoside |
| NOVATA AB (Cognis) | Cocoglycerides |
| Photosomes (AGI Dermatics) | AQUA (WATER), LECITHIN, PLANKTON EXTRACT |
| Phytokine (Coletica) | HYDROLYZED SOY PROTEIN |
| Ridulisse C (Silab) | HYDROLYZED SOY PROTEIN |
| Ronasphere LDP | SILICA, CI 77891 (TITANIUM DIOXIDE), CI 77491 (IRON OXIDES) |
| Sepivinol R (Seppic) | Wine Extract |
| Simulgel NS (Seppic) | Hydroxyethyl Acrylate / Sodium Acryloyl Dimethyltaurate Copolymer, Squalane, Polysorbate 60 |
| Tego Carbomer 140 | Carbomer |
| Ultrasomes (AGI Dermatics) | AQUA (WATER), LECITHIN, MICROCOCCUS LYSATE |

## Patentansprüche

1. Kosmetische oder dermatologische topische Zusammensetzung, enthaltend mindestens ein DNA-Reparaturenzym und mindestens ein Oligopeptid, das mindestens einen N-C₂-C₂₄-Acylrest aufweisen und/oder verestert sein kann, in einem geeigneten kosmetischen oder dermatologischen Träger.

2. Kosmetische oder dermatologische topische Zusammensetzung gemäß Anspruch 1, **dadurch gekennzeichnet, dass** das DNA-Reparaturenzym ausgewählt ist aus Photolyase und T 4 Endonuclease V sowie Mischungen dieser Enzyme.

3. Kosmetische oder dermatologische topische Zusammensetzung gemäß einem der Ansprüche 1 oder 2, **dadurch gekennzeichnet, dass** mindestens ein in Liposomen verkapseltes DNA-Reparaturenzym enthalten ist.

4. Kosmetische oder dermatologische topische Zusammensetzung gemäß einem der Ansprüche 1 - 3, **dadurch gekennzeichnet, dass** das Oligopeptid ausgewählt ist aus Di-, Tri-, Tetra-, Penta- oder Hexapeptiden, die mit mindestens einem N-C₂-C₂₄-Acylrest acyliert und/oder verestert sein können, sowie Mischungen dieser Substanzen.

5. Kosmetische oder dermatologische topische Zusammensetzung gemäß einem der Ansprüche 1 - 4, **dadurch gekennzeichnet, dass** das Oligopeptid ausgewählt ist aus Tyr-Arg und seinen N-acylierten Derivaten, insbesondere N-Acetyl-Tyr-Arg-hexyldecylester, Gly-His-Lys und seinen N-acylierten Derivaten, insbesondere N-Palmitoyl-Gly-His-Lys, Gly-His-Arg, und seinen N-acylierten Derivaten, insbesondere N-Myristoyl-Gly-His-Arg, Lys-Val-Lys und seinen N-acylierten Derivaten, insbesondere Palmitoyl-Lys-Val-Lys, Val-Tyr-Val, Gly-Gln-Pro-Arg (Rigin), Rigin-Analoga und Rigin-Derivaten, insbesondere N-Palmitoyl-Gly-Gln-Pro-Arg, Lys-Thr-Thr-Lys-Ser und seinen N-acylierten Derivaten, insbesondere N-Palmitoyl-Lys-Thr-Thr-Lys-Ser, Val-Gly-Val-Ala-Pro-Gly und seinen N-acylierten Derivaten, insbesondere N-Palmitoyl-Val-Gly-Val-Ala-Pro-Gly, Acetyl-Hexapeptide-3, Hexapeptide-4, Hexapeptide-5, Myristoyl Hexapeptide-5, Myristoyl Hexapeptide-6, Hexapeptide-8, Hexapeptide-9, Hexapeptide-10, L-Glutamylaminoethyl-indol, sowie Kombinationen dieser Substanzen, insbesondere Kombinationen aus N-Palmitoyl-Gly-His-Lys und N-Palmitoyl-Gly-Gln-Pro-Arg.

6. Kosmetische oder dermatologische topische Zusammensetzung gemäß einem der Ansprüche 1 - 5, **dadurch gekennzeichnet, dass** mindestens ein nichtionischer Emulgator mit einem HLB-Wert von 8 und darunter mit der allgemeinen Formel R¹ - O - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen ist, enthalten ist.

7. Kosmetische oder dermatologische topische Zusammensetzung gemäß einem der Ansprüche 1 - 6, **dadurch gekennzeichnet, dass** mindestens ein Wirkstoff enthalten ist, der ausgewählt ist aus
a) natürlichen Betainverbindungen,
b) Vitaminen, Provitaminen und Vitaminvorstufen der Gruppen A, B, C, E, H und K und den Estern der vorgenannten Substanzen,
c) den Flavonoiden Naringin, α-Glucosylrutin, α-Glucosylmyricetin, α-Glucosylisoquercetin, α-Glucosylquercetin, Dihydroquercetin (Taxifolin), Hesperidin, Hesperitin, Neohesperidin, Rutin, Troxerutin, Monoxerutin, Diosmin, Eriodictin und Apigenin-7-glucosid,
d) Isoflavonoiden und Isoflavonoid-reichen Pflanzenextrakten,
e) Polyphenolen und Polyphenol-reichen Pflanzenextrakten,
f) Ubichinon und Ubichinol sowie deren Derivaten,
g) Silymarin,
h) natürlich vorkommenden Xanthin-Derivaten, ausgewählt aus Coffein, Theophyllin, Theobromin und Aminophyllin,
i) Ectoin,
j) Kreatin,
k) Olivenblattextrakten, Ursolsäure, Oleanol und/oder Oleanolsäure,
l) Mono- und Polyhydroxystilbenen und deren Estern,
m) Derivaten von methyliertem Silanol,
n) Phytinsäure,
o) sowie Desoxyzuckern oder Desoxyzucker-Bausteine enthaltenden Polysacchariden.

8. Kosmetische oder dermatologische topische Zusammensetzung gemäß einem der Ansprüche 1 - 7, **dadurch gekennzeichnet, dass** sie in einen Flockstift-Applikator abgefüllt ist.

9. Verwendung einer kosmetischen oder dermatologischen topischen Zusammensetzung gemäß einem der Ansprüche 1 - 8 zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut.

10. Nicht-therapeutisches Verfahren zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut, das **dadurch gekennzeichnet ist, dass** eine kosmetische oder dermatologische topische Zusammensetzung gemäß einem der Ansprüche 1 - 8 auf die Haut aufgetragen wird.
